# EUROPEAN PATENT APPLICATION

(11) **EP 2 404 626 A2**
(43) Date of publication of application: **11.01.2012**
(21) Application number: 11250527.6
(22) Date of filing: 13.05.2011
(51) Int. Cl.: A61L 31/06, A61L 31/14

(54) **Surgical implants**

(30) Priority: 14.05.2010 US 334664 P; 29.04.2011 US 97139
(71) Applicant: Tyco Healthcare Group LP, New Haven, CT 06511 (US)
(72) Inventor: Hadba, Ahmad Robert, Middlefield, CT 06455 (US); Hodgkinson, Gerald, Guilford, CT 06437 (US)
(74) Representative: Soames, Candida Jane

(57) **Abstract**

Surgical implants include a surgical mesh which includes at least one shape memory polymer capable of transitioning the surgical mesh between a first configuration and a second configuration.

## Description

### CROSS-REFERENCE TO RELATED APPLICATIONS

This application claims the benefit of, and priority to, U.S. Provisional Application No. 61/334,664, filed on May 14, 2010, the entire disclosure of which is incorporated by reference herein.

### BACKGROUND

### Technical Field

The present disclosure relates to surgical implants, and more particularly, to surgical implants useful for hernia repair which include a mesh containing a shape memory material. In embodiments, the shape memory material is capable of transitioning the mesh between a first configuration and a second configuration.

### Description of Related Art

A hernia is a protrusion of tissue or part of an organ through injured muscle tissue or an injured membrane by which the tissue or organ is normally contained. Hernias are principally repaired by pushing back, or "reducing", the herniated tissue, and then reinforcing the defect in the injured muscle tissue, for example by an implant, such as a hernia patch. The implant may be either placed over the defect (anterior repair) or under the defect (posterior repair).

When used in laparoscopic procedures, the implant itself may be rolled, folded, or crumpled for delivery through a cannula, thereby becoming entangled and difficult to unroll at the site of implantation.

It would desirable to provide an implant for hernia repair, which may be rolled or folded prior to implantation and may unroll or unravel itself upon implantation for placement in situ.

### SUMMARY

The present disclosure includes a surgical mesh containing a plurality of filaments including at least one shape memory filament selected from the group consisting of polydioxanone, polylactide, poly(trimethylene carbonate) and copolymers thereof, wherein the shape memory filament transitions the mesh from a first configuration to a second configuration. In embodiments, the shape memory filament includes a block copolymer of polydioxanone and polylactide, wherein the polydioxanone may be present in an amount from about 5 mol% to about 20 mol% of the copolymer and the polylactide may be present in an amount from about 80 mol% to about 95 mol% of the copolymer. In other embodiments, the shape memory filament includes a block copolymer of poly(trimethylene carbonate) and polylactide, wherein the poly(trimethylene carbonate) may be present in an amount from about 5 mol% to about 20 mol% of the copolymer and the polylactide may be present in an amount from about 80 mol% to about 95 mol% of the copolymer.

The first configuration of the mesh may be selected from the group consisting of rolled, folded, crumpled, curled, and/or furled; and the second configuration may be selected from the group consisting of unrolled, uncurled, unfolded, unfurled, and/or uncrumpled. In embodiments, the second configuration may be generally flat. The shape memory filament transitions the mesh from the first configuration to the second configuration in situ, and in some embodiments, at about 37°C.

In embodiments, the mesh includes a plurality of filaments which may be knit, braided, non-woven, or woven together. The mesh may further include a grip member or a bioactive agent. Further, the mesh may be a monofilament or multifilament mesh.

Additionally, a method of making a surgical mesh is disclosed herein. The method includes incorporating at least one shape memory filament into a plurality of yarns; incorporating the plurality of yarns containing the shape memory filament into the surgical mesh; and conditioning the at least one shape memory filament to transition the mesh between a first configuration and a second configuration. The conditioning step may further include inserting the mesh in a first configuration into a mold and heating the mesh to a permanent temperature of from about 20°C to about 40°C. The first configuration may be a generally rolled, crumpled, curled, folded or furled configuration; and the second configuration may be a generally unrolled, uncrumpled, uncurled, unfolded, unfurled or flat configuration.

Further, meshes of the present disclosure may include a plurality of yarns in a 2-dimensional or 3-dimensional construct.

### BRIEF DESCRIPTION OF THE DRAWINGS

Various embodiments of the present disclosure will be discussed in more detail below in conjunction with selected embodiments and the appended drawings wherein:

FIG. 1A shows a perspective view of one embodiment of a surgical mesh in a first configuration in accordance with the principles of the present disclosure;

FIG. 1B shows a perspective view of the surgical mesh of FIG. 1A in a second configuration;

FIG. 2A illustrates a perspective view of another embodiment of a surgical mesh in accordance with the principles of the present disclosure;

FIG. 2B shows a side view of the surgical mesh of FIG. 2A in a first configuration;

FIG. 2C shows a side view of the surgical mesh of FIG. 2A in a second configuration;

FIG. 3A shows a perspective view of another embodiment of a surgical mesh in accordance with the principles of the present disclosure;

FIG. 3B shows a side view of the surgical mesh of FIG. 3A with grip members positioned substantially parallel to the substrate; and

FIG. 3C shows a side view of the surgical mesh of FIG. 3A with grip members extending outwardly from the substrate.

### DETAILED DESCRIPTION

Implants in accordance with the present disclosure include a biocompatible mesh having at least one shape memory material capable of transitioning between a first configuration and a second configuration. In embodiments, the shape memory material may be interwoven with the mesh. In particular, the shape memory material transitions the mesh from a first, rolled, crumpled, curled, furled, and/or folded configuration to a second, unrolled, unfurled, generally flat configuration.

The biocompatible mesh can be in any form that has sufficient strength to reinforce a defect in tissue. Some non-limiting examples include knitted, braided, woven, or non-woven fibrous structures. These various forms may be used alone or in combination with one another. Further, the mesh may be combined with a porous or non-porous film, foam, or gel sheet. Where temporary support (i.e., stiffness) of the tissue defect is needed, a bioabsorbable material may be used to form all or part of the mesh. Where permanent support of the tissue defect is needed, the mesh may be made entirely, or in part, of a non-bioabsorbable material. A combination of bioabsorbable and non-bioabsorbable materials may also be used to form the mesh.

The implants described herein may be manufactured out of any biocompatible bioabsorbable or non-bioabsorbable material. As used herein, the term "biodegradable" includes both bioabsorbable and bioresorbable materials. By biodegradable, it is meant that the materials decompose, or lose structural integrity under body conditions (e.g., enzymatic degradation, hydrolysis) or are broken down (physically or chemically) under physiologic conditions in the body (e.g., dissolution) such that the degradation products are excretable or absorbable by the body.

Some non-limiting examples of bioabsorbable materials which may be used to form the mesh include to polymers such as aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

More specifically, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable biodegradable polymers include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Additionally, synthetically modified natural polymers such as cellulose and polysaccharide derivatives including alkyl celluloses, hydroxyalkyl celluloses, cellulose ethers, cellulose esters, nitrocelluloses, and chitosan may be utilized. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses".

Suitable non-bioabsorbable materials include: polyolefins such as polyethylene and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers and copolymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof. Additionally, non-biodegradable polymers and monomers may be combined with each other to create a core of a filament, for example a filament possessing a core-sheath configuration. Copolymers (block or random) and mixtures and blends of such biocompatible polymeric or copolymeric materials may also be useful.

The meshes described herein may include porous fabrics made from intertwined filaments. The filaments may extend horizontally and vertically in a manner which produces sections where the filaments cross-over one another creating points of common intersection. In some embodiments, the filaments may be knitted to form two-dimensional or three-dimensional meshes. The filaments may be monofilaments or multi-filaments and, in embodiments, a plurality of multi-filaments may be combined to form yarns. It is envisioned that the mesh substrate may be configured to any size and/or shape suitable for hernia repair. The filaments may have core/sheath constructs.

Suitable meshes for use in the present disclosure include, for example, a collagen composite mesh such as PARIETEX^{™}Composite Mesh (commercially available from Tyco Healthcare Group LG, d/b/a Covidien). PARIETEX^{™} Composite Mesh is a 3-dimensional polyester weave with a resorbable collagen film bonded on one side. Another suitable mesh includes Parietex Progrip™ self-fixating mesh (also commercially available from Covidien). Parietex Progrip™ is a polyester mesh which includes poly lactic acid (PLA) microgrips. Other suitable meshes include those sold under the names PARIETENE®, PERMACOL™, PARIETEX™, SURGIPRO™ (all commercially available from Covidien); PROLENE™ (commercially available from Ethicon, Inc.); MARLEX®, DULEX®, 3D MAX® mesh, PERFIX® plug, VENTRALEX®, and KUGEL® patch (all commercially available from C.R. Bard, Inc.); PROLITE™, PROLITE ULTRA™ (all commercially available from Atrium Medical); COMPOSIX®, SEPRAMESH®, and VISILEX® (all commercially available from Davol, Inc.); and DUALMESH®, MYCROMESH®, and INFINIT® mesh (all commercially available from W.L. Gore). Additionally, meshes within the scope and context of this disclosure may include biologic materials such as allografts, autografts, and xenografts.

According to one embodiment of the present disclosure, Parietex Pro-grip™ self-fixating mesh may be employed. Pro-grip™ mesh includes a knit having a monofilament sheet forming, on one face of the knit, spiked/barbed grip members which protrude perpendicularly with respect to the sheet. The grip members each have a substantially rectilinear body and, at the free end of this body, a head of greater width than that of the body. The grip members function as hooks, which are capable of being fastened either to another prosthetic fabric (belonging to the same prosthesis or not) or directly to the biological tissues. In certain embodiments, the fabric or mesh may include grip members on at least one surface of the mesh.

Meshes may be formed using any method suitable for forming fibrous structures, including but not limited to knitting, weaving, knipling, tatting, non-woven techniques, wet-spinning, electro-spinning, gel-spinning, extrusion, co-extrusion, and the like. Suitable techniques for making mesh are within the purview of those skilled in the art.

In embodiments, two-dimensional mesh substrates may be formed as described in U.S. Patent No. 7,331,199, the entire contents of which is incorporated by reference herein. In other embodiments, the mesh substrate may be formed by knitting a three-dimensional fabric. In embodiments, three-dimensional mesh substrates may be found in U.S. Patent No. 7,021,086, U.S. Patent No. 6,596,002, and U.S. Patent No. 7,331,199, the entire contents of which are incorporated by reference herein.

Meshes of the present disclosure include a plurality of filaments containing shape memory polymers. Shape memory polymers are a class of polymers that, when formed into an object such as a filament can be temporarily deformed by mechanical force and then caused to revert back to an original shape when stimulated by energy. Shape memory polymers exhibit shape memory properties by virtue of at least two phase separated micro domains in their microstructure. The first domain is composed of hard, covalently cross-linked or otherwise chain motion-limiting structures, which act as anchors to retain the object's original shape. The second domain is a switchable soft structure, which can be deformed and then fixed to obtain a secondary or temporary shape.

In the case of heat stimulated shape memory polymers, a transition temperature (T_{Trans}) exists at which the shape change occurs during heating. The shape memory polymers can thus be tailored by altering material properties at the molecular level and by varying processing parameters.

It is envisioned that the polymeric filaments may be conditioned as shape memory polymers in the presence, or the absence, of the mesh. In embodiments, the shape memory filaments may be conditioned prior to being incorporated into the mesh. In other embodiments, the shape memory filaments may be conditioned after being incorporated into the mesh. In still other embodiments, the shape memory filaments may be attached to a separate fabric, such as a flap mesh substrate, and the separate fabric, which includes the shape memory filaments, may be attached to the biocompatible substrate by any suitable method, including, but not limited to, adhesives and stitching.

More specifically, the shape memory polymers may include filaments manufactured in a generally unfurled, unrolled, or unfolded configuration. That is to say, the permanent shape of the shape memory monofilaments is configured to enable a biocompatible substrate, such as a mesh, to lay substantially flat against tissue. The unrolled polymeric filaments may be heated above the polymer's glass transition temperature (T_{g}), which allows the polymer to become soft, flexible, and easier to mold. While above the T_{g}, the unrolled polymeric filaments may be reconfigured into a rolled or folded configuration. The reconfigured rolled or folded polymeric filaments may then be cooled below the T_{g} of the polymer to keep the filaments in the rolled/folded configuration, i.e., the temporary shape. In such embodiments, the rolled/folded polymeric filaments may be conditioned to return to the permanent form, i.e., an unrolled/unfolded configuration upon exposure to the proper external stimulus, such as a change in temperature above the T_{g} of the given polymer. It is envisioned that the filament may be incorporated into the mesh in the temporary shape. For example, the shape memory polymer may be knit, woven, or braided into a two or three dimensional architecture of the mesh.

Alternatively, a molding process may be utilized to produce the filaments of the present disclosure. Plastic molding methods include, but are not limited to, melt molding, solution molding, injection molding, extrusion molding, and compression molding. Filaments may be combined to create a mesh, the filaments not yet having shape memory characteristics imparted thereto. The mesh may be inserted into the mold in a first configuration which may be rolled, crumpled, furled, folded, or otherwise entangled. Once placed in the mold with suitable dimensions, the polymeric material used to form the mesh may be heated to a specified temperature, referred to as the permanent temperature (Tₚₑᵣₘ) which may, in embodiments, be above the T_{g} of the shape memory polymeric material utilized to form the filament. Heating of the mesh may be at suitable temperatures including, for example, from about 40°C to about 180°C, in embodiments, from about 20°C to about 55°C, for a period of time from about 2 minutes to about 60 minutes, in embodiments, from about 15 minutes to about 20 minutes, to obtain the temporary shape and dimensions. The mesh may then be removed from the mold, having shape memory characteristics imparted to the filaments, set in the first configuration. Upon reaching body temperature, the mesh will unroll or unfold to the second configuration to lay substantially flat against a tissue surface.

The temperature for deformation treatment of the mesh molded with a previously memorized shape is one that makes possible ready deformation without producing cracks and should not exceed the temperature adopted for the shape memorization (e.g., Tₚₑᵣₘ). Deformation treatment at a temperature exceeding that for the permanent shape memorization may cause the object to memorize/program a new deformed shape.

After the filament with the desired shape has been formed, the filament may be deformed above Tₜᵣₐₙₛ to obtain an alternate, temporary shape. Suitable temperatures for deformation will vary depending on the shape memory polymer utilized, but generally may be above the transition temperature of the polymer (Tₜᵣₐₙₛ), but below the Tₚₑᵣₘ. In embodiments, the shape memory polymer may be cooled from its Tₚₑᵣₘ to a lower temperature which remains above the Trans and deformed, in embodiments by hand and/or mechanical means. In other embodiments, the filament may be deformed at room temperature (about 20°C to about 25°C) to obtain its temporary shape, although the temperature may differ depending upon the particular polymer employed. The filament may then be cooled to a temperature below the Tₜᵣₐₙₛ of the material utilized to form the filament, at which time the filament of the present disclosure may be incorporated into a mesh. As the Tₜᵣₐₙₛ is usually greater than room temperature, cooling to room temperature may be sufficient to lock in the temporary shape. In order to keep the shape of the filament in its temporary shape, the shape memory mesh of the present disclosure should be stored at a temperature which will not cause a transition to the primary shape.

Once the object is heated to T>T_{Trans}, the soft segments soften and relax back to their original configuration and the object returns to its primary or original shape, sometimes referred to herein, as its permanent shape when the shape memory material reaches Tₚₑᵣₘ.

Suitable shape memory polymeric materials which may be utilized to fashion an grip members include: polyurethanes; poly(styrene-butadiene) block copolymers; polynorbornenes; caprolactones; dioxanones; diol esters including oligo (epsilon caprolactone) diol; lactic acid; lactide; glycolic acid; glycolide; ether-ester diols including oligo (p-dioxanone) diol; carbonates including trimethylene carbonate; combinations thereof; and the like. In embodiments, the shape memory polymer may be a copolymer of two components with different thermal characteristics, including: oligo (epsilon-caprolactone) dimethacrylates and butyl acrylates including poly(epsilon-caprolactone) dimethacrylate-poly (n-butyl acrylate); or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, No. 7, pp. 29-30 (July 2002), the entire disclosure of which is incorporated by reference herein.

In other embodiments, blends of materials may be utilized as the shape memory polymeric material including: urethanes blended with lactic acid and/or glycolic acid; homopolymers thereof or copolymers thereof; and acrylates blended with caprolactones such as polycaprolactone dimethacrylate poly(butyl acrylate) blends; and combinations thereof.

Other examples of these shape memory polymers and methods for forming permanent and temporary shapes therewith are set forth in Lendlein et al., "Shape Memory Polymers as Stimuli-Sensitive Implant Materials," Clinical Hemorheology and Microcirculation, 32 (2005) 105-116, and Lendlein et al., "Biodegradable, Elastic Shape-Memory Polymers for Potential Biomedical Applications," Science, Vol. 269 (2002) 1673-1676, the entire disclosures of each of which are incorporated by reference herein.

Table 1 below further illustrates compositions which demonstrate shape memory effects. The block copolymers of each composition are in annealed wire format, the proposed soft and hard segments, and the glass transition temperature (T_{g}), having been measured by differential scanning calorimetry which is equal to T_{Trans}.

**TABLE 1**

| Composition (mol%) | Soft Domain | Hard Domain | Tg (T_{Trans}) [°C] |
|---|---|---|---|
| 15% Polydioxanone 85% Poly (L-lactide) | Polydioxanone and Amorphous Polylactide | Crystalline Polylactide | 54 |
| 20% Polydioxanone 80% Poly (L-lactide) | Polydioxanone and Amorphous Polylactide | Crystalline Polylactide | 45 |
| 15% Trimethylene Carbonate 85% Poly (L-lactide) | Trimethylene Carbonate and Amorphous Polylactide | Crystalline Polylactide | 54 |
| 20% Trimethylene Carbonate 80% Poly (L-lactide) | Trimethylene Carbonate and Amorphous Polylactide | Crystalline Polylactide | 55 |

The copolymers in Table 1 may undergo a partial shift when approaching T_{g}, and T_{Trans} may be depressed when the materials are in aqueous solution. Since these polymers degrade by water absorption and bulk hydrolysis, water molecules entering the polymer matrices may act as plasticizers, causing the soft segments to soften at lower temperatures than in dry air. Thus, polymers exhibiting T_{Trans} depression in aqueous solution may maintain a temporary shape through temperature excursions in the dry state, such as during shipping and storage, and shape shift to its permanent shape at body temperatures upon implantation.

Thus, in embodiments, the shape memory polymer may include a block copolymer of polydioxanone and polylactide with the polydioxanone present in an amount from about 5 mol% to about 20 mol% of the copolymer, in embodiments from about 15 mol% to about 19 mol% of the copolymer, and the polylactide present in an amount from about 80 mol% to about 95 mol% of the copolymer, in embodiments from about 81 mol% to about 85 mol% of the copolymer. In other embodiments, the shape memory polymer may include a block copolymer of poly(trimethylene carbonate) and polylactide, with the poly(trimethylene carbonate) present in an amount from about 5 mol% to about 20 mol% of the copolymer, in embodiments from about 15 mol% to about 19 mol% of the copolymer, and the polylactide present in an amount from about 80 mol% to about 95 mol% of the copolymer, in embodiments from about 81 mol% to about 85 mol% of the copolymer.

It is envisioned that T_{Trans} may be tailored by changing block segment molar ratios, polymer molecular weight, and time allowed for hard segment formation. In embodiments, T_{Trans} may be tailored by blending various amounts of low molecular weight oligomers of the soft segment domain into the copolymer. Such oligomers may segregate to soft domains and act as plasticizers to cause a downward shift in T_{Trans}.

In some embodiments, grip member(s) may be made of a shape memory material. Suitable shape memory materials include shape memory polymers and shape memory alloys. A variety of shape memory polymers and shape memory alloys, which may be formed into filaments useful as the grip member of the present implant, include, but are not limited to, those disclosed herein.

Similarly, in other embodiments, electrically active polymers, also known as electroactive polymers, which can alter their configuration upon application of electricity, may be utilized to fashion the grip members to secure a mesh within the body. Suitable examples of electroactive polymers include: poly(aniline); substituted poly(aniline)s; polycarbazoles; substituted polycarbazoles; polyindoles; poly(pyrrole)s; substituted poly(pyrrole)s; poly(thiophene)s; substituted poly(thiophene)s; poly(acetylene)s; poly(ethylene dioxythiophene)s; poly(ethylenedioxypyrrole)s; poly(p-phenylene vinylene)s; and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in embodiments an electroactive polymer may undergo a change in shape upon the application of electricity from a low voltage electrical source (such as a battery). Electricity may also be utilized, in embodiments, to promote the change in shape of a shape memory alloy such as Nitinol. Suitable amounts of electricity which may be applied to effect such change will vary with the electroactive polymer or shape memory alloy utilized, but can be from about 5 volts to about 30 volts, in embodiments, from about 10 volts to about 20 volts. The application will result in the grip member(s) constructed of the electroactive polymer to change its shape to a gripping structure capable of gripping the mesh to the tissue at the site of an incision.

Shape memory alloys may be spun into filaments that can be used to form the biocompatible mesh substrate. Suitable materials include, but are not limited to, nitinol (NiTi), CuZnAl, CuAlNi, and FeNiAl. Methods for forming filaments from shape memory alloys are within the purview of those skilled in the art.

While an electroactive polymer does not have the same permanent shape and temporary shape as those terms are described above with respect to shape memory polymers, as used herein the term "permanent shape" as applied to an electroactive polymer may refer to, in embodiments, the shape the electroactive polymer adopts upon the application of electricity, and the term "temporary shape" as applied to an electroactive polymer may refer to, in embodiments, the shape the electroactive polymer adopts in the absence of electricity.

In other embodiments, the shape memory polymer may be a light activated shape memory polymer. Light activated shape memory polymers (LASMP) may use processes of photo-crosslinking and photo-cleaving to change the glass transition temperature (T_{g}). Photo-crosslinking may be achieved by using one wavelength of light, while a second wavelength of light reversibly cleaves the photo-crosslinked bonds. The effect achieved may be that the material may be reversibly switched between an elastomer and a rigid polymer. Light may not change the temperature, but may change the cross-linking density within the material. For example, polymers containing cinnamic groups may be fixed into predetermined shapes by UV light illumination (> 260 nm) and then recover their original shape when exposed to UV light of a different wavelength (< 260 nm). Non-limiting examples of photoresponsive switches include cinnamic acid and cinnamylidene acetic acid.

Although specifically recited as a filament, it is envisioned that the shape memory polymers may also be formed from a plurality of monofilaments, multi-filaments, and yarns formed by a plurality of multi-filaments. The shape memory filaments may be combined with any variety of other biocompatible materials to form the shape memory mesh.

In some embodiments, the shape memory polymer may display a T_{g} ranging from about 20°C to about 40°C. In such embodiments, the shape memory filament will transition from the temporary shape to the permanent shape when exposed to temperatures in this range.

In some embodiments, the biocompatible mesh substrate containing the shape memory filaments may possess a first, rolled/folded configuration at a temperature less than about 20°C and may assume an unrolled/unfolded configuration when exposed to a temperature ranging from about 20°C to about 40°C. In certain embodiments, the shape memory material may exhibit a transitional temperature at about 37°C or about body temperature. Although the shape memory filaments are disclosed predominantly as transitioning from rolled/folded to unrolled/unfolded, it is well within the scope of the present disclosure to include shape memory filaments that may transition from unrolled/unfolded to rolled/folded configurations. In addition, the actual shape of the biocompatible substrate may vary and is meant to include any possible dimension and design. The rolled/folded and unrolled/unfolded configurations may change as deemed necessary by the type of tissue in which the substrate is being implanted. In general, the shape memory filament may assume any shape suitable for providing tissue support.

In addition to providing tissue support and tissue ingrowth, the implant may further be used for delivery of a bioactive agent. Thus, in some embodiments, at least one bioactive agent may be combined with the mesh substrate, shape memory filament(s), or both. The agents may be physically admixed with the biocompatible materials used to form the substrate or filaments, coated onto the substrate or filaments, or tethered to the biocompatible materials used to form the substrate or filaments through any variety of chemical bonds. In these embodiments, the present implant may also serve as a vehicle for delivery of the bioactive agent.

The implant may be coated with or include additional bioactive agents. The term "bioactive agent," as used herein, is used in its broadest sense and includes any substance or mixture of substances that have clinical use. Consequently, bioactive agents may or may not have pharmacological activity per se, e.g., a dye. Alternatively, a bioactive agent could be any agent which provides a therapeutic or prophylactic effect; a compound that affects or participates in tissue growth, cell growth, and/or cell differentiation; an anti-adhesive compound; a compound that may be able to invoke a biological action such as an immune response; or could play any other role in one or more biological processes. It is envisioned that the bioactive agent may be applied to the implant in any suitable form of matter, e.g., films, powders, liquids, gels and the like.

In embodiments in which the substrate or filaments includes a biocompatible polymer that include a multi-arm polyethylene glycol (PEG) or PEG star (i.e., multi-branched molecule containing PEG units), the bioactive agent may be incorporated into the core of the PEG, the arms of the PEG, or combinations thereof. In embodiments, the bioactive agent may be attached to a reactive group in the PEG chain. The bioactive agent may be bound covalently, non-covalently, i.e., electrostatically, through a thiol-mediated or peptide-mediated bond, or using biotin-avidin chemistries, and the like.

Examples of classes of bioactive agents which may be utilized in accordance with the present disclosure include, for example, anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes. It is also intended that combinations of bioactive agents may be used.

In some embodiments, the bioactive agent may include bupivacaine hydrochloride, bupivacaine, or capsaicin.

Anti-adhesive agents can be used to prevent adhesions from forming between the mesh and the surrounding tissues opposite the target tissue. In addition, anti-adhesive agents may be used to prevent adhesions from forming between the implantable medical device and the packaging material. Some examples of these agents include, but are not limited to, hydrophilic polymers such as poly(vinyl pyrrolidone), carboxymethyl cellulose, hyaluronic acid, polyethylene oxide, poly vinyl alcohols, and combinations thereof.

Suitable antimicrobial agents which may be included as a bioactive agent include, for example, triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; cephalosporins; and combinations thereof. In addition, antimicrobial proteins and peptides such as bovine lactoferrin and lactoferricin B may be included as a bioactive agent.

Other bioactive agents include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents, which may be included in the substrate and/or filaments include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, RNAi; oligonucleotides; polynucleotides; and ribozymes.

Other examples of suitable bioactive agents include Avastin®, Herceptin®, Rituxan®, Tarceva®, Xeloda®, ACETMRA®, Lucentis®, Raptiva®, Rituxan®, Xolair®, Boniva®, Cathflo® Activase®, Nutropin®, Nutropin AQ®, TNKase®, Fuzeon®, Incirase®, Pegasys®, Pulmozyme®, Tamiflu®, Valcyte®, Anaprox®, Cytovene®, EC-Naprosyn®, Fansidar®, Gantrisin®, Klonopin®, Kytril®, Naprosyn®, Rocephin®, Roferon®-A, Romazicon®, Tictid®, Valuim®, Vesanoid®, Xenical®, Zenapax®, all commercially available from Genentech.

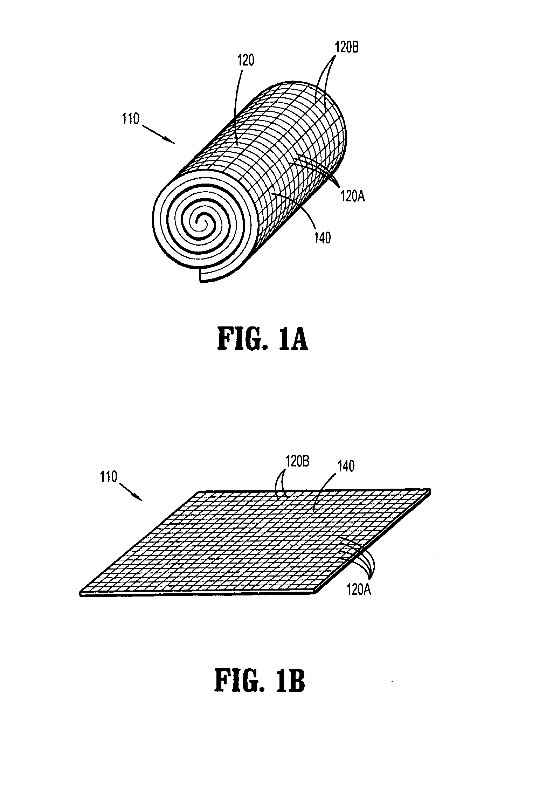

Turning now to the illustrative embodiment of FIGS. 1A and 1B, the surgical mesh 110 includes several filaments 120 which may be knit together. The filaments 120 include shape memory filaments 120a and filaments which do not possess shape memory characteristics and/or shape memory materials 120b. As illustrated, all of the shape memory filaments 120a run in the same direction and all of the non-shape memory filaments 120b run in a direction perpendicular to shape memory filaments 120a. The knit construct of the mesh 110 creates pores 140 or spaces in between filaments 120a and 120b.

As shown in FIG. 1A, mesh 110 is illustrated in a first configuration, rolled into a tubular configuration. As previously described, the temporary shape of the shape memory filaments 120a maintains the mesh 110 in the first, rolled configuration for insertion into tissue. During a laparoscopic procedure, mesh 110 may be passed through a cannula (not shown) in the rolled configuration prior to implantation.

After implantation, upon reaching the programmed transition temperature (i.e., body temperature), the shape memory filaments 120a unroll the mesh 110 into the second configuration as illustrated in FIG. 1B. The shape memory filaments 120a resume their permanent/programmed shape and the mesh 110 is positioned generally flat against a tissue surface (not shown). It should be understood that the shape memory materials may be conditioned or manufactured to transition at specific temperatures, such as, for example body temperature (about 37°C).

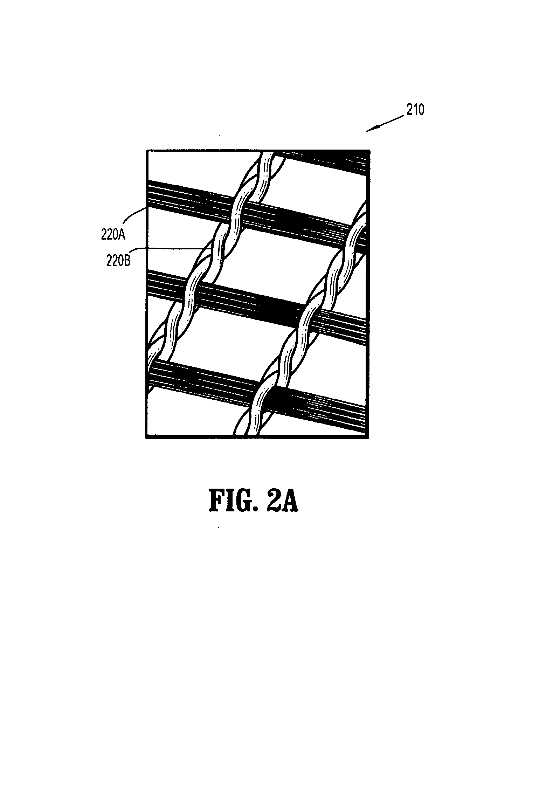

Another embodiment of a mesh according to the present disclosure is illustrated in FIGS. 2A-2C. FIG. 2A illustrates an exemplary embodiment of a multi-filament surgical mesh 210. The multi-filament mesh 210 includes a first and a second plurality of yarns, 220a and 220b, respectively. The first plurality of yarns 220a includes several filaments in a parallel configuration, while the second plurality of yarns 220b includes several filaments in a braided configuration. The first and second plurality of yarns 220a, 220b are knit together to create the multi-filament mesh 210.

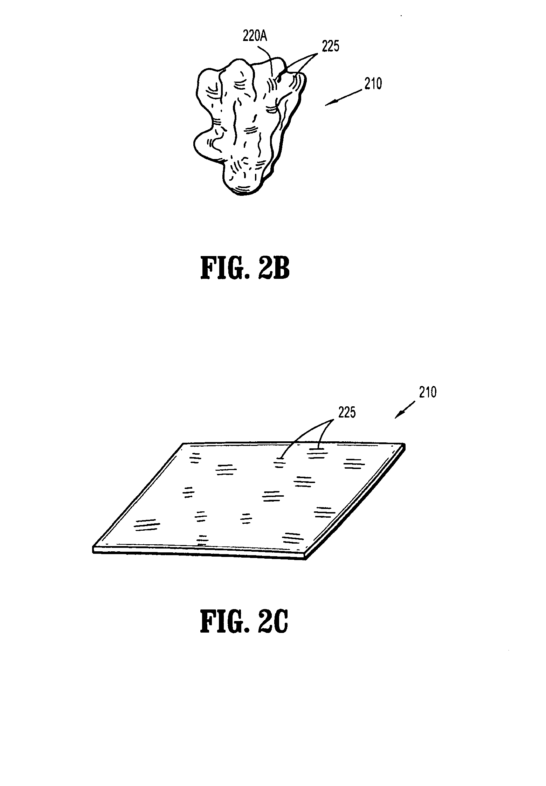

Figure 2B illustrates the multi-filament mesh 210 in a first, crumpled configuration. The first plurality of yarns 220a includes shape memory filaments 225 along at least a portion thereof. As illustrated, shape memory filaments 225 are present along several select portions of the plurality of yarns 220a. The shape memory filaments 225 are intertwined or woven with other filaments (which do not possess shape memory characteristics) such that the shape memory filaments do not extend across the entire length of the mesh, but rather extend across select portions of the mesh 210. As the shape memory filaments 225 are spread out over several portions of the mesh 210, the shape memory filaments 225 may uniformly retract the mesh 210, keeping the mesh 210 in the first crumpled configuration.

Upon insertion in situ, the shape memory filaments 225 expand the mesh 210 to the second, generally flat configuration as illustrated in FIG. 2C. The shape memory filaments 225 resume their permanent/programmed shape and the mesh 210 is positioned generally flat against a tissue surface.

In an alternate embodiment, implants in accordance with the present disclosure may include at least one grip member capable of transitioning between a first non-gripping configuration and a second gripping configuration. In the non-gripping configuration, the grip member is unable to engage any portion of the substrate or surrounding tissue upon implantation. While in the gripping configuration, the grip member is able to either attach at least a portion of the substrate to tissue and/or attach a first portion of the substrate to a second portion of the substrate.

The grip member may be made from at least one filament or yarn. In embodiments, a plurality of grip members may be positioned over an entire surface of the biocompatible substrate. In other embodiments, a plurality of grip members may be positioned over only a portion of the surface of the biocompatible substrate. In still other embodiments, the grip members may be positioned on more than one portion of the biocompatible substrate.

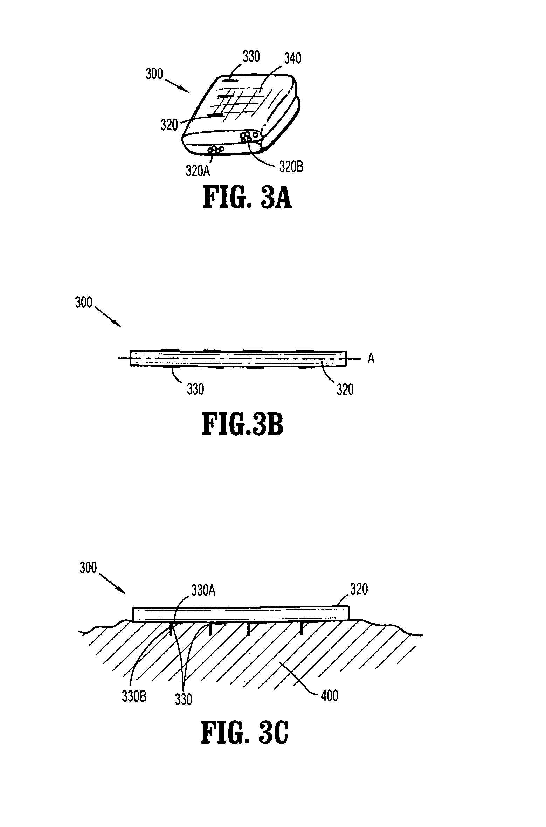

Turning to FIGS. 3A-3C, a mesh having grip members is illustrated. The surgical mesh 300 includes a plurality of filaments 320 which may be knit together. The plurality of filaments includes shape memory filaments 320a and filaments which do not possess shape memory characteristics and/or shape memory materials 320b. It is also envisioned that the configuration of the shape memory filaments 320a within the mesh may vary as a results of the first configuration. The knitted construct of the mesh 300 creates pores 340 or spaces in between the filaments 320a and 320b.

The mesh 300 also includes grip members 330 which are illustrated in a non-gripping position in FIGS. 3A and 3B. The grip members 330, made of a shape memory material conditioned to transition above a temperature of about 20°C, will transition from a non-gripping configuration to a gripping configuration (FIG. 3C). Grip members 330 are in a generally straight manner positioned parallel to the longitudinal axis (A) of substrate 300 (FIG. 3B). Because the grip members run generally parallel to the axis of mesh 300, grip members 330 are unable to penetrate into the surrounding tissue upon implantation or penetrate into other portions of mesh 300. In the non-gripping position, grip-members 330 are unable to attach to other portions of mesh 300 and are also unable to attach to the surrounding tissue upon implantation. Although shown as generally parallel to the longitudinal axis (A) of mesh 300, grip members 330 do not have to be generally parallel. In embodiments, the grip members may be in any position relative to the longitudinal axis of the substrate which may be suitable for preventing the grip members from attaching to the tissue and/or other portions of the substrate.

As shown in FIG. 3A, mesh 300 is illustrated in a first, folded configuration. As previously described, the temporary shape of the shape memory filaments 320a maintains the mesh 300 in a first, folded configuration for insertion into tissue. The grip members 330 are in a non-gripping position prior to exposure to a predetermined stimulus (i.e., a specific programmed temperature). The first, folded configuration facilitates insertion through a laparoscopic/endoscopic port or cannula (not shown).

After implantation, upon exposure to a predetermined stimulus, (i.e., the programmed transition temperature which may be about body temperature/37°C), the shape memory filaments 320a unfold the mesh 300 into the second, unfolded configuration as illustrated in FIG. 3B. The shape memory filaments 320a resume their permanent/programmed shape and the mesh 300 is positioned generally flat against a tissue surface. Additionally, upon insertion of the mesh, grip members 330 will transition from the non-gripping configuration to a gripping configuration following exposure to the stimulus, such as an increase in temperature to between about 20°C and about 40°C. As depicted in FIG. 3C, grip members 330 may form a generally L-shaped configuration wherein base member 330a of grip member 330 is positioned within a portion of mesh 300 and arm member 330b of grip member 330 extends generally perpendicular from the longitudinal axis of substrate 300 to attach mesh 300 to tissue 400.

Methods of making a surgical implant with tissue adherent properties are disclosed above. The methods may further include conditioning a grip member that extends from a biocompatible substrate to assume a first, non-gripping configuration, and upon exposure to a stimulus assume a second, gripping configuration.

Persons skilled in the art will understand that the devices and methods specifically described herein, and illustrated in the accompanying drawings, are non-limiting exemplary embodiments. It is envisioned that the elements and features illustrated or described in connection with one exemplary embodiment may be combined with the elements and features of another without departing from the scope of the present disclosure. It will be understood that various modifications may be made to the embodiments disclosed herein. For example, in embodiments, the implant may include additional layers of materials such as an antimicrobial coating layer or a film for preventing adhesion formation on a portion of the substrate. Thus, those skilled in the art will envision other modifications within the scope and spirit of the claims.
The invention may be described by reference to the following numbered paragraphs:-
1. A surgical mesh comprising a plurality of filaments including at least one shape memory filament selected from the group consisting of polydioxanone, polylactide, poly(trimethylene carbonate) and copolymers thereof, wherein the shape memory filament transitions the surgical mesh from a first configuration to a second configuration.
2. The surgical mesh of paragraph 1, wherein the shape memory filament comprises a block copolymer of polydioxanone and polylactide.
3. The surgical mesh of paragraph 2, wherein the polydioxanone is present in an amount from about 5 mol% to about 20 mol% of the copolymer and the polylactide is present in an amount from about 80 mol% to about 95 mol% of the copolymer.
4. The surgical mesh of paragraph 1, wherein the shape memory filament comprises a block copolymer of poly(trimethylene carbonate) and polylactide.
5. The surgical mesh of paragraph 4, wherein the poly(trimethylene carbonate) is present in an amount from about 5 mol% to about 20 mol% of the copolymer and the polylactide is present in an amount from about 80 mol% to about 95 mol% of the copolymer.
6. The surgical mesh of paragraph 1, further comprising a grip member.
7. The surgical mesh of paragraph 1, wherein the shape memory filament transitions the mesh from the first configuration to the second configuration at about 37°C.
8. The surgical mesh of paragraph 1, wherein the first configuration is selected from the group consisting of rolled, folded, crumpled, curled, furled, and combinations thereof.
9. The surgical mesh of paragraph 1, wherein the second configuration is selected from the group consisting of unrolled, uncurled, unfolded, unfurled, uncrumpled, and combinations thereof.
10. The surgical mesh of paragraph 1, wherein the second configuration is generally flat.
11. The surgical mesh of paragraph 1, wherein the plurality of filaments is knit, braided, non-woven or woven.
12. The surgical mesh of paragraph 1, further comprising a bioactive agent.
13. The surgical mesh of paragraph 1, wherein the mesh comprises a monofilament or multifilament mesh.
14. A method of making a surgical mesh, the method comprising incorporating the at least one shape memory filament into a plurality of yarns; incorporating the plurality of yarns containing the shape memory filament into the surgical mesh; and conditioning the at least one shape memory filament to transition the mesh between a first configuration and a second configuration.
15. The method of paragraph 14, wherein the conditioning step further includes inserting the mesh in a first configuration into a mold and heating the mesh to a permanent temperature of from about 20°C to about 55°C.
16. The method of paragraph 14, wherein the first configuration comprises a generally rolled, crumpled, curled, folded, or furled configuration.
17. The method of paragraph 14, wherein the mesh transitions from the first configuration to the second configuration in situ.
18. The method of paragraph 14, wherein the second configuration comprises a generally unrolled, uncrumpled, uncurled, unfolded, unfurled, or flat configuration.
19. The method of paragraph 18, wherein the at least one shape memory filament comprises a permanent shape in the second configuration.
20. The method of paragraph 14, wherein the mesh comprises the plurality of yarns in a 2-dimensional or 3-dimensional construct.

## Claims

1. A surgical mesh comprising:
a plurality of filaments including at least one shape memory filament selected from the group consisting of polydioxanone, polylactide, poly(trimethylene carbonate) and copolymers thereof, wherein the shape memory filament transitions the surgical mesh from a first configuration to a second configuration.

2. The surgical mesh of claim 1, wherein the shape memory filament comprises a block copolymer of polydioxanone and polylactide; preferably wherein the polydioxanone is present in an amount from about 5 mol% to about 20 mol% of the copolymer and the polylactide is present in an amount from about 80 mol% to about 95 mol% of the copolymer.

3. The surgical mesh of claim 1 or claim 2, wherein the shape memory filament comprises a block copolymer of poly(trimethylene carbonate) and polylactide, preferably wherein the poly(trimethylene carbonate) is present in an amount from about 5 mol% to about 20 mol% of the copolymer and the polylactide is present in an amount from about 80 mol% to about 95 mol% of the copolymer.

4. The surgical mesh of any preceding claim, further comprising a grip member.

5. The surgical mesh of any preceding claim, wherein the shape memory filament transitions the mesh from the first configuration to the second configuration at about 37°C.

6. The surgical mesh of any preceding claim, wherein the first configuration is selected from the group consisting of rolled, folded, crumpled, curled, furled, and combinations thereof; and/or wherein the second configuration is selected from the group consisting of unrolled, uncurled, unfolded, unfurled, uncrumpled, and combinations thereof, preferably wherein the second configuration is generally flat.

7. The surgical mesh of any preceding claim, wherein the plurality of filaments is knit, braided, non-woven or woven.

8. The surgical mesh of any preceding claim, further comprising a bioactive agent.

9. The surgical mesh of any preceding claim, wherein the mesh comprises a monofilament or multifilament mesh.

10. A method of making a surgical mesh, the method comprising:
incorporating the at least one shape memory filament into a plurality of yarns;
incorporating the plurality of yarns containing the shape memory filament into the surgical mesh; and
conditioning the at least one shape memory filament to transition the mesh between a first configuration and a second configuration.

11. The method of claim 10, wherein the conditioning step further includes inserting the mesh in a first configuration into a mold and heating the mesh to a permanent temperature of from about 20°C to about 55°C.

12. The method of claim 10 or claim 11, wherein the first configuration comprises a generally rolled, crumpled, curled, folded, or furled configuration.

13. The method of any of claims 10 to 12, wherein the mesh transitions from the first configuration to the second configuration in situ.

14. The method of any of claims 8 to 13, wherein the second configuration comprises a generally unrolled, uncrumpled, uncurled, unfolded, unfurled, or flat configuration.

15. The method of any of claims 8 to 14, wherein the at least one shape memory filament comprises a permanent shape in the second configuration; and/or wherein the mesh comprises the plurality of yarns in a 2-dimensional or 3-dimensional construct.
